# EUROPEAN PATENT APPLICATION

(11) **EP 1 502 947 A1**
(43) Date of publication of application: **02.02.2005**
(21) Application number: 04017937.6
(22) Date of filing: 29.07.2004
(51) Int. Cl.: C12N 1/20, C12N 11/04, A23C 21/02

(54) **Use of proteins from whey for the production of microorganisms**

(30) Priority: 01.08.2003 IT MI20031593
(71) Applicant: Proge Farm S.r.l., 28100 Novara (IT)
(72) Inventor: Dondi, Glancaria, 28065 Cerano (NO) (IT); Malfa, Patrizia, 28100 Novara (IT)
(74) Representative: Valentini, Giuliano

(57) **Abstract**

The present invention relates to the use of whey for the protection of probiotic and non- probiotic microorganisms, methods for treatment of said microorganisms, the microorganisms so obtained and pharmaceutical and nutraceutical compositions containing them.

## Description

The present invention concerns the use of whey for the microorganisms protection, in particular probiotic microorganisms, a method for the protection of said microorganisms, microorganisms so treated and pharmaceutical and nutraceutical formulations that containing them.

The aim of this invention is to obtain probiotic microorganisms, able to resist to process stresses (fermentation, concentration, freeze-dried, etc.) as well as to persist alive at room temperature during the shelf-life period.

The residual from whey dairy is one of the products derived from dairy industry which, although is containing noble proteins an other precious components, it presents relevant employment and disposal problems.

Every year, in Italy, about 7 millions of tons of whey are produced in more than 2500 dairies, spread all over the country.

The disposal of whey obtained from milk (by now called only "whey") in the dairy industry is a very important problem for the environment, because of it has a very strong polluting amount (COD 70.000 ppm of O₂).

For this reasons, the Italian dairies face a difficult environmental emergency, due to technical-economical difficulties for the dairying refuses draining away and for whey, in particular.

It stands out the need of new technologies, that are able to recover the different components of the whey, to be used in some field such as food, dietetic, nutraceutical, pharmaceutical and cosmetic field.

Up today the most important application for the residual whey of diary productions has been in the animal and human food; in fact the whey is a complete food under a proteic point of view. Its components, fragmented or alone, can represent an important energy source; beside, scientifically, it has been demonstrated that they have the following properties:
they improve the immune defences,
they decrease cholesterol,
they are involved as coadjuvants to decrease the cancer frequency,
they have anti-oxidative properties.

Actually the whey elements are used in the so called "functional product".

The expression "functional product/food" means to group all the products where different elements (proteins, vitamins, fats and carbohydrates) are mixed in the optimal way, to support any lacks much better if compared to natural combinations.

It has been surprisingly found out that it is possible employ noble whey proteins, present into the whey itself derived by dairy processes, for the treatment and the protection, of probiotic or not probiotic strains.

A probiotic strain, according to the widest means accepted by scientific community, is a microorganism that, if it is ingested in certain numbers exerts health benefits for the host.

The most used strains in functional foods or in pharmaceutical formulations can be divided into lactic or not lactic bacteria. A lot of these strains have got as feature to be thermo-sensible. It is necessary to obtain probiotic microorganisms which can be stored at room temperature. In fact stability tests, made in such conditions, showed a large mortality of microorganisms. Instead, these phenomena are considerably decreased when the storing conditions are in cold temperatures.

The use of concentrate whey and/or its fractions in the different process steps allows to recycle a lot of dairy whey, avoiding its draining away that is a great ecologic and economic problem and, in the meantime, protecting the probiotic microorganisms.

In fact, the present invention allows to produce probiotic microorganisms (bacteria) which are resistant to the process stresses, giving a protection that also remains during the following shelf-life period.

In fact, very good results have been obtained using whey proteins, either in the fermentation step (addition, in the standard medium, of whey-proteins as a growth promoter), or during the freeze-dried step and following micro-encapsulation made by a thin film (addition of whey-proteins as protectors).

So, according to one of its aspects, the invention concerns the use of whey for the treatment of probiotic microorganisms.

The term " microorganisms" according to the present invention designates either probiotic and non-probiotic microorganisms. Probiotic microorganisms are particularly preferred according to the invention.

Whey, concentrated whey and whey-proteins optionally isolated and re-suspended in aqueous solution, if it is necessary are all suitable to be used according to the invention.

So, according to one of its aspects, the present invention concerns the use of whey as a growth promoter and/or protective factor for probiotic microorganisms.

In fact, it has been observed that adding whey to the probiotic microorganisms, in the culture medium, the probiotic growth is improved.

According to one of its different aspects, the present invention concerns a method to protect and/or improve the probiotic microorganism growth, comprising adding to the growth medium some whey, preferably concentrated, in amount comprised between 10% and 50% by volume with respect to the growth medium of probiotics.

Alternatively, it is possible to directly add whey-proteins, in this case the amount is advantageously up to 50% with respect to the concentrated whey.

According to another of its aspect, the present invention concerns a method to protect and/or improve probiotic microorganism growth that includes to add some whey, preferably concentrated, or some whey-proteins to the probiotic microorganisms during the dehydration steps which are conventionally carried out.

In this way a micro-encapsulation of probiotic microorganisms is performed.

The micro-encapsulation technique, using whey-proteins, has got remarkable advantages compared to the techniques used until now because it is natural and safe.

Preferably, before submitting the microorganisms to the method according to the present invention, probiotic bacteria are pre-treated using conventional methods allowing preservation of strains until their use.

According to a preferred realisation way, probiotic bacteria are treated with whey or with derived products thereof during the different processing steps.

For example, during the fermentation step, probiotic microorganisms are incubated in growth substrates supplied with whey-proteins through either addition of concentrated whey or isolated whey-proteins. The so obtained bacteria are centrifuged and re-suspended in a medium containing particular additives such as whey-proteins acting as protective agents during the dehydration step ( freeze-dryer, spray-dryer, etc.).

Alternatively, according to a micro-encapsulation technique that uses the method of the present invention, an emulsion of dry bacteria with a solution of whey-proteins is prepared first. Then the emulsion is submitted to a drying treatment through the use of a conventional spray-dryer, that permits the formation of a powder made by spherical particles (micro-capsules), inside of which probiotic bacteria are present.

The instant process allows to obtain dried probiotic microorganisms able to resist at room temperature during the shelf-life period. The good results of this method also depend on the selected probiotic strain but, in any case, the result is always better than when the method is not used.

## Claims

1. A method of treatment of cultures of microorganisms **characterized in that** concentrated whey or isolated whey-proteins are added to said cultures.

2. Method according to claim 1, **characterized in that** said microorganisms are probiotic microorganisms.

3. Method according to claims 1 or 2, **characterized in that** concentrated whey or isolated whey-proteins are added to the growth medium of the microorganisms.

4. Method according to claim 3, **characterized in that** concentrated whey is added in a amount comprised between 10% and 50% by volume with respect to the growth medium of the microorganisms.

5. Method according to claim 3, **characterized in that** whey-proteins are added in an amount which is up to 50% of the concentrated whey.

6. Method according to claims 1 or 2, **characterized in that** concentrated whey or isolated whey-proteins are added to the microorganisms during the dehydrating step.

7. Method according to claim 6, **characterized in that** that a bacteria suspension is mixed with whey or with a whey-proteins mixture and then the emulsion so obtained is submitted to a dehydrating treatment.

8. Probiotic and non-probiotic microorganism cultures, treated with the method according to one of claims 1 to 7 and probiotic and non-probiotic microorganism micro-encapsulated with the method of claims 1 to 7.

9. Pharmaceutical, nutraceutical, dietary and food compositions containing one or more strains treated according to claims 1-7, mixed with optional excipients.

10. The use of whey or derivatives thereof as a protective agent, growth promoter or for the micro-encapsulation of microorganisms.
